Europäisches Patentamt

(19) European Patent Office

Office européen des brevets

(11) Publication number: **0 064 529**
**B1**

(12) EUROPEAN PATENT SPECIFICATION

(45) Date of publication of patent specification: **29.07.87**

(51) Int. Cl.⁴: **A 61 F 5/44**

(21) Application number: **81903127.9**

(22) Date of filing: **09.11.81**

(86) International application number:
**PCT/EP81/00177**

(87) International publication number:
**WO 82/01648 27.05.82 Gazette 82/14**

(54) URINE COLLECTING DEVICE.

(30) Priority: **17.11.80 NL 8006266**

(43) Date of publication of application:
**17.11.82 Bulletin 82/46**

(45) Publication of the grant of the patent:
**29.07.87 Bulletin 87/31**

(84) Designated Contracting States:
**AT CH DE FR GB LI LU NL SE**

(56) References cited:
**US-A-3 604 424**
**US-A-3 721 243**
**US-A-3 835 857**
**US-A-4 020 843**

**Pamphlet "Urihesive", distributed at 14th
Congress Dt. Ges. F. Biomedizinische Technik
field in BERLIN from 10 to 12 September 1980
by Von Heyden CONVATEC (SQUIBB)**

(73) Proprietor: **Cuijkse Medische Centrale B.V.
Korte Oijen 2
Nl-5433 NE Cuijk (NL)**

(72) Inventor: **Haan, Wilhelmus Albertus Otto
Bazuinlaan 7
NL-5402 PB Uden (NL)**

(74) Representative: **Hoorweg, Petrus Nicolaas et al
OCTROOIBUREAU ARNOLD & SIEDSMA
Sweelinckplein 1
NL-2517 GK The Hague (NL)**

Courier Press, Leamington Spa, England.

## Description

The invention relates to a device for collecting urine from incontinent male patients, which comprises an external catheter, a urine receptacle and a flexible tube connecting the catheter to the receptacle, in which the catheter comprises a thin flexible cylindrical portion and, connected thereto, a generally conical portion which is stiffer than the cylindrical portion and which terminates in a discharge opening, and a lining adapted to be applied to the penis of the patient and to secure the cylindrical portion of the catheter thereof.

Urine collecting devices are known or have been described in US—A—3,835,857 which comprise an external catheter, a urine receptacle and a flexible tube connecting the catheter to the receptacle and further a lining adapted to be applied to the penis of the patient to secure the external catheter thereto. Such devices normally incorporate a non-return valve between the catheter and the receptacle. The external catheters of such devices are not entirely satisfactory from the point of view of patient comfort and obtrusiveness under the patient's clothing.

I have now developed a urine collecting device of this type which includes a novel form of catheter and, in preferred embodiments of the device, other features which considerably increase the comfort of the user and reduce the obtrusiveness of the device under the user's clothing.

According to the present invention, there is provided a device for collecting urine from incontinent male patients, which is distinguished in that the axis of the cylindrical portion of the catheter forms an angle with the axis of the conical portion and in that the lining is in the form of a strip having a length and width such that when wound helically in overlapped configuration on the penis it extends the greater part of the length of the cylindrical portion.

The generally conical portion of the catheter need not necessarily taper towards the discharge opening in a straight line and the taper may be generally rounded. This portion of the catheter should be positioned, in use, so as to accommodate the glans of the user's penis and its relative stiffness or rigidity and its dimensions are such that the walls of this portion are normally spaced from the surface of the glans.

The angling of this portion with respect to the cylindrical portion of the catheter ensures better flow of urine to the discharge opening when the user is lying down and thus reduce the likelihood of contact between urine and the penis which can lead to irritation. This feature also reduces the obtrusiveness of the device under the user's clothing.

It is noticed that a collecting device as disclosed in US—A—3,604,424, comprises a cylindrical receiving container, enveloping a rubber tube and being provided with a conical portion; said conical portion being angled with respect to said cylindrical container.

The lining is preferably formed of a textile material having some elasticity. The lining strip is preferably coated with a non-irritant adhesive on both sides, but, in order to reduce irritation to the user, particularly when such a device has to be used for long periods, it is preferred that at least one surface of the lining strip should be free of adhesive over an area extending from one end of the strip and/or that the adhesive coating on at least one surface of the strip should be non-continuous.

Further features and advantages of the present invention will appear from the following description of preferred embodiments thereof, given by way of example only, with reference to the accompanying drawings, in which:

Fig. 1 is a diagrammatic side view, partly in section, of an embodiment of the invention,

Fig. 2 is a plan view of a lining strip, and

Fig. 3 is a diagrammatic axial section of another embodiment of the catheter.

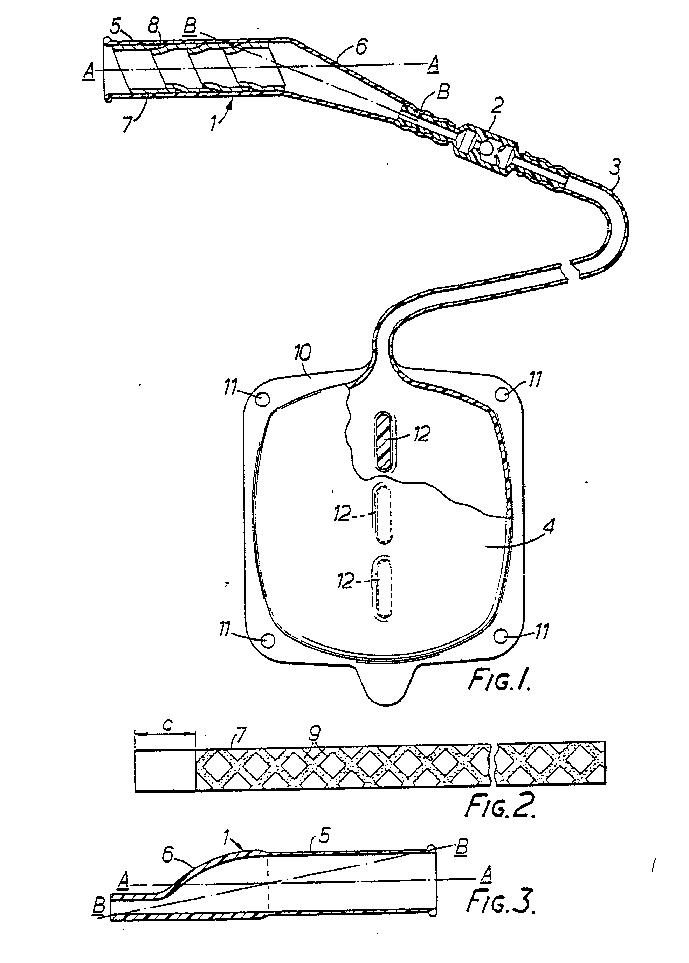

The device shown in Fig. 1 comprises a catheter 1, the discharge opening of which is connected to a non-return valve 2 which, in turn, is connected to a flexible tube 3 which leads to a urine receptacle 4.

The catheter 1 comprises a flexible cylindrical portion 5 which is sufficiently thin that it can be fitted easily to the shaft of the user's penis. The cylindrical portion 5 leads to a conical portion 6, the centre line A of the portion 5 forming an angle with the centre line of the portion 6. When the catheter is correctly fitted to the patient, this angle ensures that urine flows to the valve 2 even when the patient is lying down and that no pool or drops of urine remain in the catheter to cause irritation to the penis.

To improve the attachment of the catheter to the penis, a lining strip 7 is provided which is of such a length and width that it can be wound helically on the shaft of the user's penis and, when so wound, occupies substantially the whole of the length of the cylindrical portion 5 of the catheter when the latter is then fitted. The spirally wound strip 7 is shown in Fig. 1 and the strip itself in Fig. 2. It will be noted that such helical winding of the strip 7 leaves a helical channel 8 between the wound strip and the wall of the cylindrical portion. This channel 8 serves to provide an air passage between the outside and the conical portion 6 of the catheter thus ensuring that negative pressure, which might interfere with proper flow of urine out of the catheter, cannot be created in the conical portion 6 when the device is in use.

The strip is preferably coated with adhesive on both sides, but in order to reduce irritation over prolonged periods of use, the adhesive coating on at least the surface which is applied to the penis may be non-continuous. Fig. 2 shows the strip 7 provided with criss-cross lines of adhesive 9. Alternatively or in addition a portion C at one end of the strip may be left free of adhesive altogether.

The urine receptacle 4 shown in Fig. 1 is a bag of flexible plastics material. The peripheral edge 10 of the bag is welded together and is furnished with openings 11 for ties to attach it, for example, to the leg of the patient. The walls of the bag 4 lying opposite each other are attached to each other at 12 so that free passage of the liquid into the reservoir is ensured, but splashing is counteracted. Naturally the points of attachment can be made in a form other than the rectangular welds shown in Fig. 1.

Fig. 3 shows an alternative form of catheter 1 in which similar parts have been given the same reference numerals and letters as in Fig. 1. In this embodiment the taper of the conical portion 6 is distinctly rounded. This catheter is made as a unitary dipped latex article, extra thickness of latex being provided in the conical portion to provide the stiffness required.

## Claims

1. A device for collecting urine from incontinent male patients, which comprises an external catheter (1), a urine receptacle (4) and a flexible tube (3) connecting the catheter (1) to the receptacle (4), in which the catheter comprises a thin flexible cylindrical portion (5) and, connected thereto, a generally conical portion (6) which is stiffer than the cylindrical portion and which terminates in a discharge opening, and a lining (7) adapted to be applied to the penis of the patient to secure the cylindrical portion (5) of the catheter (1) thereto, characterized in that the axis (A—A) of the cylindrical portion (5) of the catheter (1) forms an angle with the axis (B—B) of the conical portion (6) and in that the lining (7) is in the form of a strip having a length and width such that when wound helically in overlapped configuration on the penis it extends the greater part of the length of the cylindrical portion (5).

2. A device according to claim 1, characterized in that the catheter (1) is formed as a single moulded or dipped article which is of greater thickness, and therefore stiffness, in the generally conical portion (6).

3. A device according to claim 1, characterized in that the lining strip (7) is coated with a non-irritant adhesive on both sides.

4. A device according to claim 3, characterized in that at least one surface of the lining strip (7) is free of adhesive over an area extending from one end thereof.

5. A device according to claims 3 or 4, characterized in that the adhesive coating on at least one surface of the lining strip (7) is non-continuous.

6. A device according to any of claims 1 to 5, characterized in that the urine receptacle (4) is a bag of flexible plastics material, the opposed walls of the bag being attached to one another at a plurality of spaced points (12).

## Patentansprüche

1. Vorrichtung zum Sammeln von Urin dauernässender männlicher Patienten, mit einem externen Katheter (1), einem Urinaufnahmegefäß (4) und einem biegsamen Schlauch (3), der den Katheter (1) mit dem Aufnahmegefäß (4) verbindet, wobei der Katheter einen dünnen, biegsamen zylindrischen Abschnitt (5) und damit verbunden einen insgesamt konischen Abschnitt (6) aufweist, der steifer als der zylindrische Abschnitt ist und in einer Ausflußöffnung mündet und einer Einlage (7), die so bemessen ist, daß sie auf den Penis des Patienten paßt, um den zylindrischen Abschnitt (5) des Katheters (1) daran zu befestigen, dadurch gekennzeichnet, daß die Achse (A—A) des zylindrischen Abschnitts (5) des Katheters (1) einen Winkel mit der Achse (B—B) des konischen Abschnitts (6) bildet, und daß die Einlage (7) die Form eines Streifens hat, dessen Länge und Breite so bemessen sind, daß er über den größeren Teil der Länge des zylindrischen Abschnittes (5) reicht, wenn er überlappend schraubenlinienförmig um den Penis gewickelt ist.

2. Vorrichtung nach Anspruch 1, dadurch gekennzeichnet, daß der Katheter (1) als einzelner Guß- oder Tauchartikel ausgebildet ist, der in dem insgesamt konischen Abschnitt (6) von größerer Dicke und deswegen von größerer Steifigkeit ist.

3. Vorrichtung nach Anspruch 1, dadurch gekennzeichnet, daß der Einlagestreifen (7) beidseitig mit einem nichtreizenden Klebematerial beschichtet ist.

4. Vorrichtung nach Anspruch 3, dadurch gekennzeichnet, daß wenigstens eine Oberfläche des Einlagestreifens (7) in einem Bereich, der sich von einem Ende des Streifens aus erstreckt, frei von Klebematerial ist.

5. Vorrichtung nach Anspruch 3 oder 4, dadurch gekennzeichnet, daß die Klebeschicht auf wenigstens einer Oberfläche des Einlagestreifens (7) diskontinuierlich aufgetragen ist.

6. Vorrichtung nach einem der Ansprüche 1 bis 5, dadurch gekennzeichnet, daß das Urinaufnahmegefäß (4) ein Sack aus flexiblem Kunststoffmaterial ist, dessen gegenüberliegende Wände an einer Mehrzahl von im Abstand voneinander liegenden Stellen (12) miteinander verbunden sind.

## Revendications

1. Dispositif pour la récupération des urines de patients mâles incontinents, qui comprend un cathéter externe (1), un réceptacle à urine (4) et un tube flexible (3) reliant le cathéter (1) au réceptacle (4), où le cathéter présente une partie cylindrique flexible mince (5) et, reliée à elle, une partie généralement conique (6) qui est plus rigide que la partie cylindrique et qui se termine par une ouverture de décharge, et une doublure (7) destinée à s'adapter au pénis du patient afin d'y fixer la partie cylindrique (5) du cathéter (1), caractérisé en ce que l'axe (A—A) de la partie cylindrique (5) du cathéter (1) fait un angle avec l'axe (B—B) de la partie conique (6) et en ce que la doublure (7) a la forme d'un ruban dont la longueur et la largeur sont telles que lorsqu'il est enroulé hélicoïdalement pour recouvrir le pénis, il s'étend sur la plus grande partie de la longueur de la partie cylindrique (5).

2. Dispositif selon la revendication 1, caractérisé en ce que le cathéter (1) est fait d'une seule pièce moulée ou traitée, qui est d'épaisseur plus grande et ainsi plus rigide dans la partie généralement conique (6).

3. Dispositif selon la revendication 1, caractérisé. en ce que le ruban de doublure (7) est recouvert d'un adhésif non irritant sur les deux faces.

4. Dispositif selon la revendication 3, caractérisé en ce qu'au moins une surface du ruban de doublure (7) est dépourvue d'adhésif sur une zône s'étendant à partir d'une extrémité de celui-ci.

5. Dispositif selon l'une des revendications 3 ou 4, caractérisé en ce que la couche adhésive sur au moins une surface du ruban de doublure (7) est discontinue.

6. Dispositif selon l'une quelconque des revendications 1 à 5, caractérisé en ce que le réceptacle d'urine (4) est un sac en matériau plastique flexible, dont les parois opposées sont réunies l'une à l'autre en plusieurs points espacés les uns des autres (12).

0 064 529

FIG.1.

FIG.2.

FIG.3.